# EUROPÄISCHE PATENTSCHRIFT

(11) **EP 1 067 113 B1**
(45) Veröffentlichungstag und Bekanntmachung des Hinweises auf die Patenterteilung: **20.07.2005**
(21) Anmeldenummer: 00113066.5
(22) Anmeldetag: 26.06.2000
(51) Int. Cl.: C07C 209/58, C07C 213/02, C07C 235/34

(54) **Verfahren zur Herstellung von Phenethylaminen und neue chemische Verbindungen**
Process for the preparation of phenethylamines and intermediates therefor
Procédé pour la préparation de phénéthylamines et des intermédiaires

(30) Priorität: 06.07.1999 DE 19931116
(43) Veröffentlichungstag der Anmeldung: 10.01.2001
(73) Patentinhaber: LANXESS Deutschland GmbH, 51369 Leverkusen (DE)
(72) Erfinder: Müller, Peter, Dr., 50674 Köln (DE); Marhold, Albrecht, Dr., 51373 Leverkusen (DE)

(56) Entgegenhaltungen:
- EP-A- 0 314 422
- EP-A- 0 344 577
- DE-A- 2 704 365
- BAEK, GI HYEON ET AL: "Synthesis of 3-arylpropylamine derivatives from aryl halides using Heck reaction" BULL. KOREAN CHEM. SOC., Bd. 20, Nr. 2, 1999, Seiten 232-236, XP001006391
- MCFARLAND, JAMES W. ET AL: "Novel anthelmintic agents. V. Thiazoline and dihydrothiazine analogs of pyrantel" J. MED. CHEM. , Bd. 13, Nr. 1, 1970, Seiten 113-119, XP001002431
- LEWIS, FREDERICK D. ET AL: "Configuration-dependent photoisomerization of (E)-cinnamamides" J. AM. CHEM. SOC., Bd. 110, Nr. 15, 1988, Seiten 5191-5192, XP001002405
- HOULIHAN, WILLIAM J. ET AL: "Sleep-inducing N-alkyl-5-[m-(trifluoromethyl)phenyl]-5-hy droxy-2- pyrrolidinones and N-alkyl-3-(trifluoromethyl)cinnamamides" J. MED. CHEM. , Bd. 28, Nr. 1, 1985, Seiten 28-31, XP001002430

## Beschreibung

Die vorliegende Erfindung betrifft ein neues Verfahren zur Herstellung von Fluor enthaltenden Phenethylaminen und dabei auftretende neue chemische Verbindungen.

4-Fluorphenethylamin, 4-(Trifluormethoxy)-phenethylamin und andere fluorhaltige Phenethylamine sind Zwischenprodukte für die Herstellung von Agrochemikalien. Aus J. Am. Chem. Soc. 63, 602 (1941) ist bekannt, daß man 4-Fluorphenethylamin in einem mehrstufigen Prozeß herstellen kann. Dabei wird ausgehend von p-Fluorphenylmagnesiumbromid durch Addition von Ethylenoxid mit nachfolgender Hydrolyse p-Fluorphenethylalkohol erhalten, der mit Phosphortribromid in p-Fluorphenylethylbromid umgewandelt wird, das mit Ammoniak umgesetzt 4-Fluorphenethylamin ergibt. Nachteilig für eine Anwendung dieses Verfahrens im technischen Maßstab ist der große Aufwand, der für die Durchführung einer Grignard-Reaktion notwendig ist und die vielstufige Synthese.

Bei einem anderen Verfahren zur Herstellung von 4-Fluorphenethylamin (siehe J. Org. Chem. 23, 1979 (1958)) wird von p-Fluorbenzylchlorid ausgegangen, dieses mit Natriumcyanid zum p-Fluorphenylessigsäurenitril umgesetzt, daß dann schließlich mit Natrium- oder Lithiumalanat reduziert wird. Nachteilig bei diesem Verfahren ist die teilweise schlechte Herstellbarkeit der benötigten Benzylchloride und der für die Handhabung von Natrium- oder Lithiumalanat notwendige große sicherheitstechnische Aufwand. Dieses Verfahren ist also für eine technische Anwendung auch nicht geeignet.

Es besteht daher noch immer ein Bedürfnis nach einem Verfahren, mit dem fluorhaltige Phenethylamine auf einfache Weise, von gut zugänglichen Ausgangsprodukten und ohne hohen technischen Aufwand hergestellt werden können.

Es wurde nun ein Verfahren zur Herstellung von Fluor enthaltenden Phenethylaminen der Formel (I) gefunden 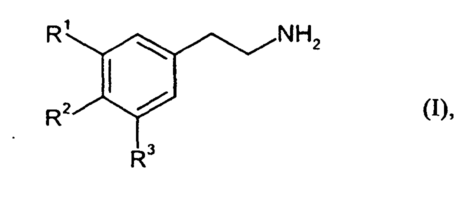 in der
von den Resten R¹ und R² einer für Fluor, Trifluormethyl, Trifluormethoxy, Tetrafluorethoxy oder Pentafluorethoxy und der andere für Wasserstoff, C₁-C₆-Alkyl, C₁-C₆-Alkoxy, Fluor, Trifluormethyl, Trifluormethoxy, Tetrafluorethoxy oder Pentafluorethoxy steht oder
R¹ und R² gemeinsam für -O-CF₂-O-, -O-CF₂-CF₂- oder -O-CF₂-CF₂-O- stehen und
R³ für Wasserstoff, Chlor, C₁-C₆-Alkyl oder C₁-C₆-Alkoxy steht,
das dadurch gekennzeichnet ist, daß man in einer ersten Stufe ein substituiertes Brombenzol der Formel 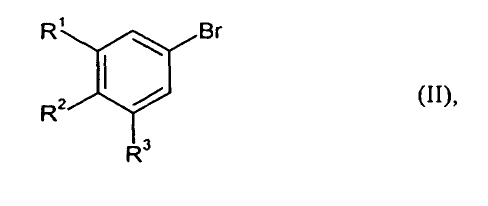 in der
R¹, R² und R³ die bei Formel (I) angegebene Bedeutung haben,
mit Acrylamid in Gegenwart eines Palladiumkatalysators umsetzt,
in einer zweiten Stufe das erhaltene Arylacrylamid der Formel 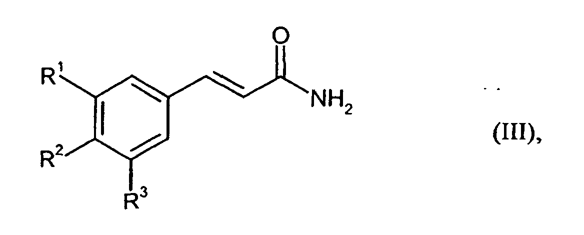 in der
die Reste R¹, R² und R³ die bei Formel (I) angegebene Bedeutung haben,
katalytisch hydriert und
in einer dritten Stufe das in der zweiten Stufe erhaltene Arylamid der Formel 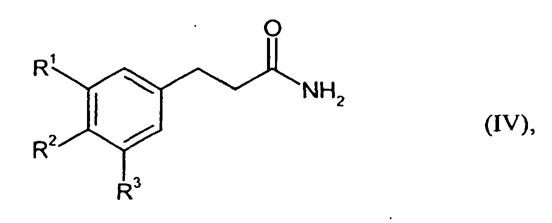 in der
R¹, R² und R³ die bei Formel (I) angegebene Bedeutung haben,
umlagert.

In den Formeln (1), (II), (III) und (IV) steht einer der Reste R¹ und R² bevorzugt für einen Rest aus der Reihe Fluor, Trifluormethyl, Trifluormethoxy, Tetrafluorethoxy oder Pentafluorethoxy und der andere Rest bevorzugt für Wasserstoff oder es stehen R¹ und R² bevorzugt gemeinsam für -O-CF₂-O-, -O-CF₂-CF₂- oder -O-CF₂-CF₂-O-. R³ steht bevorzugt für Wasserstoff oder Chlor.

Besonders bevorzugt stehen in den Formeln (I), (II), (III) und (IV) R¹ und R³ für Wasserstoff und R² für Fluor, Trifluormethyl oder Trifluormethoxy oder R¹ für Fluor, Trifluormethyl oder Trifluormethoxy und R² und R³ für Wasserstoff oder R¹ und R² gemeinsam für -O-CF₂-O-, -O-CF₂-CF₂- oder -O-CF₂-CF₂-O- und R³ für Wasserstoff.

Bei Tetrafluorethoxyresten handelt es sich vorzugsweise um 1,1,2,2-Tetrafluorethoxyreste.

Ganz besonders bevorzugt werden erfindungsgemäß die in Tabelle 1 angegebenen, Fluor enthaltenden Phenethylamine hergestellt.

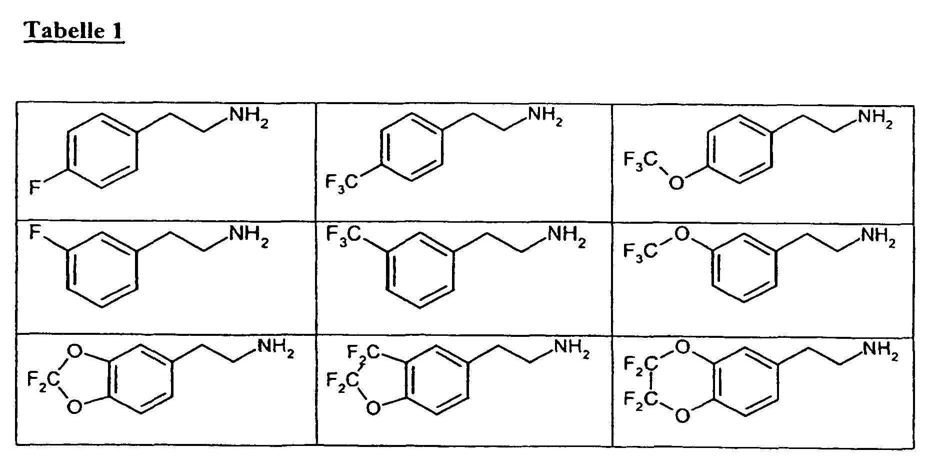

Die Umsetzung der Brombenzole der Formel (II) mit Acrylamid zu Arylacrylamiden der Formel (III) wird in Gegenwart eines Palladiumkatalysators durchgeführt, wobei vorzugsweise Verdünnungsmittel und Reaktionshilfsmittel zugegen sind.

Die Brombenzole der Formel (II) sind entweder kommerziell erhältlich oder nach an sich bekannten Verfahren oder analog dazu herstellbar.

Als Palladiumkatalysator für die erste Reaktionsstufe geeignet sind beispielsweise Palladiumkomplexe mit Aryl- oder Alkylphosphinen als Liganden. Man kann z.B. sowohl die Komplexe selbst als auch Palladium(II)-Salze und die freien Liganden separat zusetzen.

Als Verdünnungsmittel für die erste Reaktionsstufe sind dipolare, aprotische Lösungsmittel und diese enthaltende Mischungen, beispielsweise mit aliphatischen und/oder aromatischen Kohlenwasserstoffen und/oder Ethern geeignet. Beispiele für dipolare, aprotische Lösungsmittel sind: Nitrile wie Acetonitril, Propionitril, n- und i-Butyronitril und Benzonitril, Amide wie Formamid, N-Methylformamid, N,N-Dimethylformamid, N,N-Dimethylacetamid und N-Methylpyrrolidon, Ester wie Methyl-, Ethyl- und Butylacetat, Sulfone wie Sulfolan. Es können auch Mischungen von dipolaren, aprotischen Lösungsmitteln eingesetzt werden.

Als Reaktionshilfsmittel für die erste Reaktionsstufe kommen z.B. schwache anorganische oder organische Basen in Frage. Bevorzugt sind Erdalkalimetall- und Alkalimetallacetate, -carbonate und -hydrogencarbonate wie Natrium-, Kalium-, Calcium- und Ammoniumacetat, Natrium-, Kalium- und Ammoniumcarbonat, Natrium- und Kaliumhydrogencarbonat und tertiäre Amine wie Trimethylamin, Triethylamin und Tributylamin. Bevorzugt setzt man Natrium- oder Kaliumacetat ein.

Zur Durchführung der ersten Reaktionsstufe kann man z.B. das jeweilige Brombenzol der Formel (II) und Acrylamid in Mengen von 0,5 bis 2 Mol des jeweiligen Brombenzols bezogen auf 1 Mol Acrylamid einsetzen. Vorzugsweise liegt diese Menge bei 0,9 bis 1,1 Mol. Besonders bevorzugt arbeitet man mit äquimolaren Mengen des jeweiligen Brombenzols der Formel (II) und Acrylamid. Bezogen auf das jeweilige Brombenzol kann man z.B. 0,005 bis 20 mmol, bevorzugt 0,1 bis 10 mmol Palladiumkatalysator und 1 bis 10 Äquivalente, bevorzugt 1 bis 3 Äquivalente Reaktionshilfsmittel einsetzen. Wenn man getrennt ein Palladium(II)-Salz und freie Phosphinliganden einsetzt, kann das Molverhältnis von Palladium(II)-Salz zu Phosphinliganden z.B. 1:1 bis 1:10, bevorzugt 1:2 bis 1:4 betragen. Die Menge Verdünnungsmittel ist nicht kritisch. Bevorzugt setzt man pro Mol des jeweiligen Brombenzols der Formel (II) 100 bis 2000 ml ein.

Die Reaktionstemperatur in der ersten Verfahrensstufe kann in einem größeren Bereich variiert werden. Sie kann z.B. zwischen 50 und 180°C, bevorzugt zwischen 80 und 150°C liegen.

Die katalytische Hydrierung von Arylacrylamiden der Formel (III) zu Arylamiden der Formel (IV) wird i.a. mit Wasserstoffgas und vorzugsweise in Gegenwart eines Verdünnungsmittels durchgeführt.

Als Katalysator zur Durchführung dieser Hydrierung sind beispielsweise Edelmetalle auf Trägermaterialien, insbesondere Palladium und Platin auf Kohle, Silikaten, Siliciumdioxid, Aluminiumoxid, Zeolithen, Bariumsulfat, Calciumcarbonat und Spinellen geeignet. Besonders bevorzugt ist Palladium auf Kohle. Bezogen auf den fertigen Katalysator kann er z.B. 0,1 bis 20 Gew.-% Edelmetall enthalten. Vorzugsweise liegt diese Menge bei 5 bis 15 Gew.-%.

Als Verdünnungsmittel für die zweite Reaktionsstufe kommen Wasser, organische Lösungsmittel und beliebige Mischungen davon in Betracht. Beispielhaft seien als organische Lösungsmittel genannt: aliphatische, alicyclische und aromatische Kohlenwasserstoffe wie Petrolether, Hexan, Heptan, Cyclohexan, Methylcyclohexan, Benzol, Toluol, Xylol und Decalin, Ether wie Diethyl-, Diisopropyl-, Methyl-t-butyl- und Methyl-t-amylether, Dioxan, Tetrahydrofuran, 1,2-Dimethoxyethan, 1,2-Diethoxyethan, Diethylenglykoldimethylether und Anisol, Ester wie Methyl-, Ethyl- oder Butylacetat und Alkohole wie Methanol, Ethanol, n- und i-Propanol, n-, i-, s- und t-Butanol, Ethandiol, Propan-1,2-diol, Ethoxyethanol, Methoxyethanol, Diethylenglykolmonomethylether und Diethylenglykolmonoethylether. Bevorzugte Lösungsmittel sind Tetrahydrofuran, Toluol, Ethylacetat, Methyl-t-butylether und Ethanol. Besonders bevorzugt verwendet man Tetrahydrofuran.

Zur Durchführung der zweiten Reaktionsstufe kann man z.B. pro Mol Arylacrylamid der Formel (III) 0,001 bis 0,5 Mol, bevorzugt 0,05 bis 0,2 Mol Katalysator (gerechnet als Metall) und 1 bis 101 Verdünnungsmittel einsetzen. Der Katalysator kann mehrmals hintereinander eingesetzt werden. Bei Ansätzen mit bereits benutztem Katalysator kann es zur Verlängerung der Reaktionszeit kommen. Dem kann man durch Zugabe von frischem Katalysator entgegensteuern, z.B. in einer Menge von 5 bis 25 Gew.-%, bezogen auf den gesamten eingesetzten Katalysator.

Die Reaktionstemperatur für die zweite Reaktionsstufe kann in einem größeren Bereich variiert werden. Sie kann z.B. zwischen 0 und 120°C, bevorzugt zwischen 20 und 70°C liegen. Der Wasserstoffdruck kann z.B. 1 bis 100 bar, bevorzugt zwischen 5 und 20 bar betragen.

Die Umlagerung der Arylamide der Formel (IV) zu den Phenethylaminen der Formel (I) kann beispielsweise mit einer wäßrigen Base in Gegenwart von Brom oder Chlor durchgeführt werden. Als Basen sind beispielsweise wäßrige Alkalihydroxide geeignet. Bevorzugt werden wäßrige Lösungen von Natriumhydroxid oder Kaliumhydroxid eingesetzt.

Pro Mol Arylamid der Formel (IV) kann man beispielsweise 1 bis 20 Äquivalente wäßriges Alkalihydroxid und 0,5 bis 5 Äquivalente Brom oder Chlor einsetzen. Vorzugsweise setzt man 4 bis 8 Äquivalente Alkalihydroxid und 0,9 bis 1,5 Äquivalente Brom oder Chlor ein.

Die Temperatur bei der Umlagerung kann in weiten Grenzen variiert werden. Nach oben ist die Temperatur durch den Siedepunkt der Reaktionsmischung begrenzt. Vorzugsweise arbeitet man bei 50 bis 120°C.

Die Umsetzungen der ersten und dritten Stufe des erfindungsgemäßen Verfahrens können bei Normaldruck oder erhöhtem Druck durchgeführt werden. Vorzugsweise wird dabei bei Normaldruck gearbeitet.

Die Reaktionsdurchführung, Aufarbeitung und Isolierung der Reaktionsprodukte kann nach allgemein üblichen bekannten Methoden erfolgen. Die Endprodukte werden vorzugsweise durch Kristallisation, Destillation oder durch Entfernung der flüchtigen Bestandteile, gegebenenfalls im Vakuum, gereinigt.

Die vorliegende Erfindung betrifft weiterhin Arylacrylamide der Formel 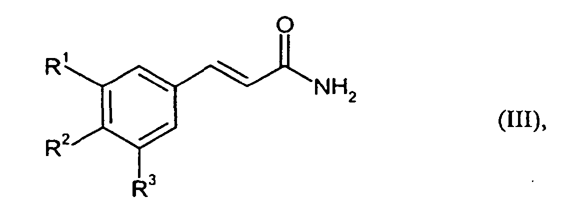 in der
von den Resten R¹ und R²einer für Fluor, Trifluormethyl, Trifluormethoxy, Tetrafluorethoxy oder Pentafluorethoxy und der andere für Wasserstoff, steht oder
R¹ und R² gemeinsam für -O-CF₂-O-, -O-CF₂-CF₂- oder -O-CF₂-CF₂-O- stehen und
R³ für Wasserstoff oder Chlor steht, mit Ausnahme der Verbindungen 3-(3-Fluorphenyl)-acrylamid, 3-[3-(Trifluormethyl)-phenyl]-acrylamid und 3-[4-(Trifluormethyl)phenyl]-acrylamid. Die bevorzugten und besonders bevorzugten Bedeutungen der Reste R¹, R² und R³ sind wie oben angegeben.

Die vorliegende Erfindung betrifft schließlich auch Arylamide der Formel 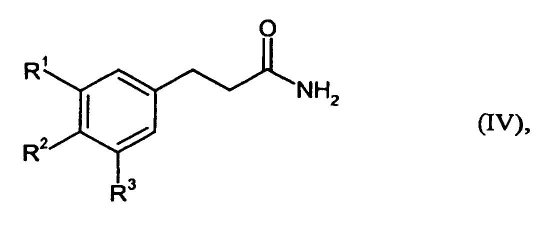 in der
von den Resten R¹ und R²einer für Fluor, Trifluormethyl, Trifluormethoxy, Tetrafluorethoxy oder Pentafluorethoxy und der andere für Wasserstoff, steht oder
R¹ und R² gemeinsam für -O-CF₂-O-, -O-CF₂-CF₂- oder -O-CF₂-CF₂-O- stehen und
R³ für Wasserstoff oder Chlor, steht,
mit Ausnahme der Verbindungen 3-(3-Fluorphenyl)-propionylamid, 3-(4-Fluorphenyl)-propionylamid, 3-[3-(Trifluormethyl)-phenyl]-propionylamid und 3-[4-(Trifluorphenyl]-propionylamid. Die bevorzugten und besonders bevorzugten Bedeutungen der Reste R¹, R² und R³ sind wie oben angegeben.

Die neuen Arylacrylamide und Arylamide können wie oben beschrieben hergestellt und als Zwischenprodukte, die nicht immer zwischenisoliert werden müssen, zur Herstellung von Fluor enthaltenden Phenethylaminen der Formel (I) verwendet werden. Die Zwischenisolierung kann insbesondere auf der Stufe der Acrylamide der Formel (IV) unterbleiben.

Mit dem erfindungsgemäßen Verfahren gelingt die Herstellung von Fluor enthaltenden Phenethylaminen in einem dreistufigen Verfahren, und es wird damit ein Verfahren zur Verfügung gestellt, das auch in technischem Maßstab auf einfache Weise durchführbar ist und so einen wesentlich günstigeren Zugang zu Fluor enthaltenden Phenethylaminen ermöglicht. Besonderer sicherheitstechnischer Aufwand ist nicht erforderlich.

Das Auffinden der erfindungsgemäßen Acrylarylamide und der erfindungsgemäßen Arylamide machte die Konzeption des erfindungsgemäßen Verfahrens im hier dargelegten Umfang erst möglich.

### Beispiele

### Beispiel 1

275 ml trockenes Dimethylformamid wurden 1 Stunde lang mit Stickstoff gespült. Dazu wurden nacheinander 100 g 1-Brom-4-(trifluormethoxy)-benzol, 29,4 g Acrylamid, 101 mg Palladium(II)-acetat, 216 mg Triphenylphosphin und 67,8 g Natriumacetat gegeben und 20 Stunden bei 130°C gerührt. Nach dem Abkühlen wurde filtriert, das Filtrat eingeengt, der Filterrückstand mit dem eingeengten Filtrat vereinigt, das Gemisch in 200 ml Wasser suspendiert und erneut filtriert. Der jetzt vorliegende Filterrückstand wurde mit 200 ml n-Hexan gewaschen und an der Luft getrocknet. Es wurden 84 g 3-[4-(Trifluormethoxy)-phenyl]-acrylamid erhalten. Das Produkt war laut GC, angegeben in Flächenprozent, 95,9 %ig. Das entspricht einer Ausbeute von 83 % der Theorie.

### Beispiel 2

In einem Rührautoklav wurden 250 g des gemäß Beispiel 1 erhaltenen 3-[4-(Trifluormethoxy)-phenyl]-acrylamid gelöst in 1250 ml Tetrahydrofuran vorgelegt, 6,2 g Palladium-auf-Kohle (10 gew.-%ig) zugesetzt und bei 50°C und einem Wasserstoffdruck zwischen 5 und 10 bar bis zum vollständigen Umsatz (3 Stunden) hydriert. Danach wurde vom Katalysator abfiltriert und das Lösungsmittel abdestilliert. Es wurden 221 g 3-[4-(Trifluormethoxy)-phenyl]-propionamid erhalten. Das Produkt war laut GC, angegeben in Flächenprozent, 98,1 %ig. Das entspricht einer Ausbeute von 86 % der Theorie.

### Beispiel 3

Es wurden 21,1 g Natriumhydroxid in 70 ml Wasser vorgelegt und bei Raumtemperatur 15 g Brom zugetropft. Nach beendeter Zugabe wurden 20 g des nach Beispiel 2 erhaltenen 3-[4-(Trifluormethoxy)-phenyl]-propionamids zugefügt. Danach wurde die Mischung 3 Stunden unter Rückfluß erhitzt. Nach dem Abkühlen wurde das Reaktionsgemisch mit 200 ml Wasser verdünnt und dreimal mit je 75 ml Methyl-t-butylether extrahiert. Die vereinigten organischen Phasen wurden über Magnesiumsulfat getrocknet, filtriert und eingeengt. Der Rückstand wurde unter vermindertem Druck über eine kurze Vigreux-Kolonne destilliert. Bei 83 bis 86°C und 11 mbar übergehend wurden 10,3 g 2-[4-(Trifluormethoxy)-phenyl]-ethylamin erhalten. Laut GC, angegeben in Flächenprozent, war es 99 %ig. Das entspricht einer Ausbeute von 58 % der Theorie.

### Beispiel 4

20 g von nach Beispiel 2 erhaltenem 3-[4-(Trifluormethoxy)-phenyl]-propionamid wurden in 30 ml Wasser vorgelegt. Zu dieser Suspension wurde bei 60°C eine Mischung aus 21,1 g Natriumhydroxid, 50 ml Wasser und 15 g Brom getropft. Anschließend wurde die Mischung 3 Stunden unter Rückfluß erhitzt. Nach Verdünnen mit 200 ml Wasser wurde das Reaktionsgemisch wie in Beispiel 3 beschrieben aufgearbeitet und gereinigt. Es wurden 8,8 g 3-[4-(Trifluormethoxy)-phenyl]-acrylamid erhalten, das bei 84 bis 87°C und 11 mbar überging. Laut GC, angegeben in Flächenprozent, war es 99 %ig. Das entspricht einer Ausbeute von 49 % der Theorie.

### Beispiel 5

Es wurden 21,1 g Natriumhydroxid und 20 g von nach Beispiel 2 hergestelltem 3-[4-(Trifluormethoxy)-phenyl]-propionamid in 70 ml Wasser vorgelegt und bei Raumtemperatur 15 g Brom zugetropft. Danach wurde die Mischung 3 Stunden unter Rückfluß erhitzt. Anschließend wurde das Reaktionsgemisch mit 200 ml Wasser versetzt und wie in Beispiel 3 beschrieben aufgearbeitet und gereinigt. Es wurden 12,9 g 2-[4-(Trifluormethoxy)-phenyl]-ethylamin erhalten, das bei 84 bis 86°C und 10 mbar überging. Das erhaltene Produkt war laut GC 99 %ig, angegeben in Flächenprozent. Das entspricht einer Ausbeute von 72 % der Theorie.

## Patentansprüche

1. Verfahren zur Herstellung von Fluor enthaltenden Phenethylaminen der Formel 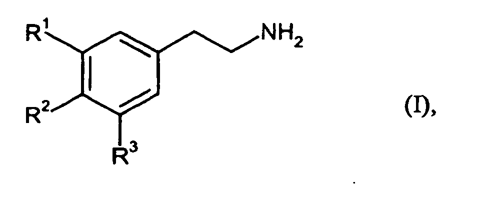 in der
von den Resten R¹ und R² einer für Fluor, Trifluormethyl, Trifluormethoxy, Tetrafluorethoxy oder Pentafluorethoxy und der andere für Wasserstoff, C₁-C₆-Alkyl, C₁-C₆-Alkoxy, Fluor, Trifluormethyl, Trifluormethoxy, Tetrafluorethoxy oder Pentafluorethoxy steht oder
R¹ und R² gemeinsam für -O-CF₂-O-, -O-CF₂-CF₂- oder -O-CF₂-CF₂-O-stehen und
R³ für Wasserstoff, Chlor, C₁-C₆-Alkyl oder C₁-C₆-Alkoxy steht,
**dadurch gekennzeichnet, daß** man in einer ersten Stufe ein substituiertes Brombenzol der Formel 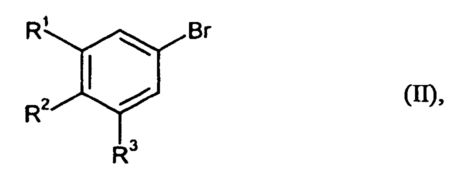 in der
R¹, R² und R³ die bei Formel (I) angegebene Bedeutung haben,
mit Acrylamid in Gegenwart eines Palladiumkatalysators umsetzt,
in einer zweiten Stufe das erhaltene Arylacrylamid der Formel 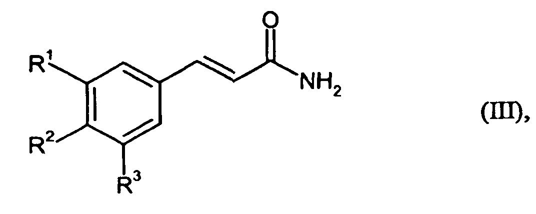 in der
die Reste R¹, R² und R³ die bei Formel (I) angegebene Bedeutung haben,
katalytisch hydriert und
in einer dritten Stufe das in der zweiten Stufe erhaltene Arylamid der Formel 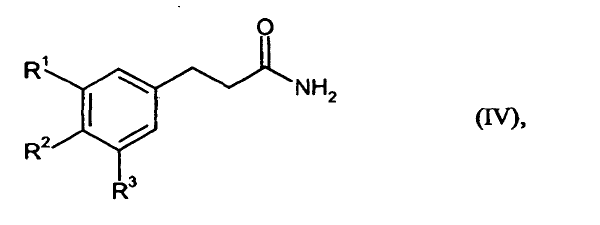 in der
R¹, R² und R³ die bei Formel (I) angegebene Bedeutung haben,
umlagert.

2. Verfahren nach Anspruch 1, **dadurch gekennzeichnet, daß** in den Formeln (I), (II), (III) und (IV) einer der Reste R¹ und R² für einen Rest aus der Reihe Fluor, Trifluormethyl, Trifluormethoxy, Tetrafluorethoxy oder Pentafluorethoxy und der andere Rest für Wasserstoff steht oder R¹ und R² gemeinsam für -O-CF₂-O-, -O-CF₂-CF₂- oder -O-CF₂-CF₂-O- stehen und R³ für Wasserstoff oder Chlor steht.

3. Verfahren nach Ansprüchen 1 und 2, **dadurch gekennzeichnet, daß** als Palladiumkatalysator für die erste Reaktionsstufe Palladiumkomplexe mit Aryl- oder Alkylphosphinen als Liganden eingesetzt werden.

4. Verfahren nach Ansprüchen 1 bis 3, **dadurch gekennzeichnet, daß** man in die erste Reaktionsstufe 0,5 bis 2 Mol des jeweiligen Brombenzols bezogen auf 1 Mol Acrylamid und 0,005 bis 20 mmol Palladiumkatalysator bezogen auf das jeweilige Brombenzol einsetzt und diese Verfahrensstufe bei 50 bis 180°C durchführt.

5. Verfahren nach Ansprüchen 1 bis 4, **dadurch gekennzeichnet, daß** man die katalytische Hydrierung von Arylacrylamiden der Formel (III) mit Wasserstoffgas und mit Edelmetallen auf Trägermaterialien als Katalysator durchführt.

6. Verfahren nach Ansprüchen 1 bis 5, **dadurch gekennzeichnet, daß** die zweite Reaktionsstufe in Gegenwart eines Verdünnungsmittels durchführt.

7. Verfahren nach Ansprüchen 1 bis 6, **dadurch gekennzeichnet, daß** man in die zweite Reaktionsstufe pro Mol Arylacrylamid der Formel (III) 0,001 bis 0,5 Mol Katalysator (gerechnet als Metall) einsetzt und diese Reaktionsstufe bei 0 bis 120°C durchführt.

8. Verfahren nach Ansprüchen 1 bis 7, **dadurch gekennzeichnet, daß** man die Umlagerung der Arylamide der Formel (IV) zu den Phenethylaminen der Formel (I) mit einer wäßrigen Base in Gegenwart von Brom oder Chlor durchführt.

9. Arylacrylamide der Formel 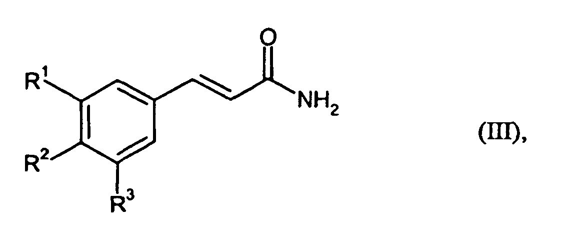 in der
von den Resten R¹ und R² einer für Fluor, Trifluormethyl, Trifluormethoxy, Tetrafluorethoxy oder Pentafluorethoxy und der andere für Wasserstoff steht oder
R¹ und R²gemeinsam für -O-CF₂-O-, -O-CF₂-CF₂- oder -O-CF₂-CF₂-O-stehen und
R³ für Wasserstoff oder Chlor steht,
mit Ausnahme der Verbindungen 3-(3-Fluorphenyl)-acrylamid, 3-[3-(Trifluormethyl)-phenyl]-acrylamid und 3-[4-(Trifluormethyl)phenyl]-acrylamid.

10. Arylamide der Formel 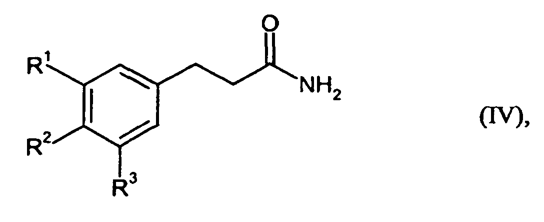 in der
von den Resten R¹ und R²einer für Fluor, Trifluormethyl, Trifluormethoxy, Tetrafluorethoxy oder Pentafluorethoxy und der andere für Wasserstoff steht oder
R¹ und R² gemeinsam für -O-CF₂-O-, -O-CF₂-CF₂- oder -O-CF₂-CF₂-O- stehen und
R³ für Wasserstoff oder Chlor steht,
mit Ausnahme der Verbindungen 3-(3-Fluorphenyl)-propionylamid, 3-(4-Fluorphenyl)-propionylamid, 3-[3-(Trifluormethyl)-phenyl]-propionylamid und 3-[4-(Trifluorphenyl]-propionylamid.

## Claims

1. Process for preparing fluorine-containing phenethylamines of the Formula 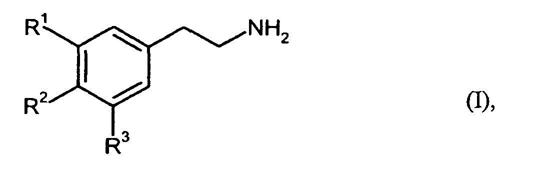 in which
one of the radicals R¹ and R² represents fluorine, trifluoromethyl, trifluoromethoxy, tetrafluoroethoxy or pentafluoroethoxy and the other represents hydrogen, C₁-C₆-alkyl, C₁-C₆-alkoxy, fluorine, trifluoromethyl, trifluoromethoxy, tetrafluoroethoxy or pentafluoroethoxy or
R¹ and R² together represent -O-CF₂-O-, -O-CF₂-CF₂- or -O-CF₂-CF₂-O- and
R³ represents hydrogen, chlorine, C₁-C₆-alkyl or C₁-C₆-alkoxy,
**characterized in that** in a first step a substituted bromobenzene of the Formula 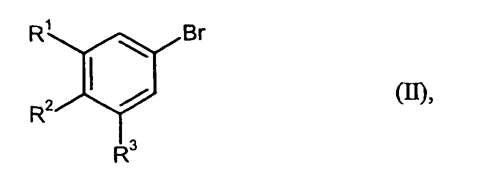 in which
R¹, R² and R³ are as defined under Formula (I)
is reacted with acrylamide in the presence of a palladium catalyst,
in a second step the resulting arylacrylamide of the Formula 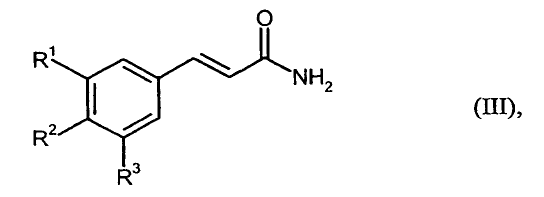 in which
the radicals R¹, R² and R³ are as defined under Formula (I)
is hydrogenated catalytically and
in a third step the arylamide obtained in step two of the Formula 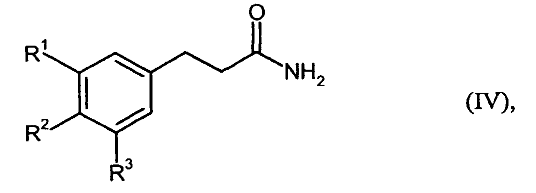 in which
R¹, R² and R³ are as defmed under Formula (I)
is rearranged.

2. Process according to Claim 1, **characterized in that** in the Formulae (I), (II), (III) and (IV) one of the radicals R¹ and R² represents a radical from the group consisting of fluorine, trifluoromethyl, trifluoromethoxy, tetrafluoroethoxy and pentafluoroethoxy and the other radical represents hydrogen or R¹ and R² together represent -O-CF₂-O-, -O-CF₂-CF₂- or -O-CF₂-CF₂-O- and R³ represents hydrogen or chlorine.

3. Process according to Claim 1 or 2, **characterized in that** the palladium catalysts used for the first reaction step are palladium complexes having aryl- or alkylphosphines as ligands.

4. Process according to Claims 1 to 3, **characterized in that** in the first reaction step from 0.5 to 2 mol of the bromobenzene in question, based on 1 mol of acrylamide, and from 0.005 to 20 mmol of palladium catalyst, based on the bromobenzene in question, are used, and that this process step is carried out at from 50 to 180°C.

5. Process according to Claims 1 to 4, **characterized in that** the catalytic hydrogenation of arylacrylamides of the Formula (III) is carried out using hydrogen gas and noble metals on support materials as catalyst.

6. Process according to Claims 1 to 5, **characterized in that** the second reaction step is carried out in the presence of a diluent.

7. Process according to Claims 1 to 6, **characterized in that** in the second reaction step, from 0.001 to 0.5 mol of catalyst (calculated as metal) are used per mole of arylacrylamide of the Formula (III), and that this reaction step is carried out at from 0 to 120°C.

8. Process according to Claims 1 to 7, **characterized in that** the rearrangement of the arylamides of the Formula (IV) to the phenethylamines of the Formula (I) is carried out using an aqueous base in the presence of bromine or chlorine.

9. Arylacrylamides of the Formula 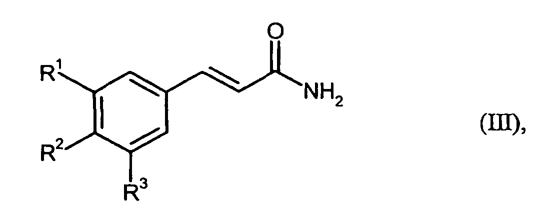 in which
one of the radicals R¹ and R² represents fluorine, trifluoromethyl, trifluoromethoxy, tetrafluoroethoxy or pentafluoroethoxy and the other represents hydrogen or
R¹ and R² together represent -O-CF₂-O-, -O-CF₂-CF₂- or -O-CF₂-CF₂-O- and
R³ represents hydrogen or chlorine,
with the exception of the compounds 3-(3-fluorophenyl)acrylamide, 3-[3-(trifluoromethyl)phenyl]acrylamide and 3-[4-(trifluoromethyl)-phenyl]acrylamde.

10. Arylamides of the Formula 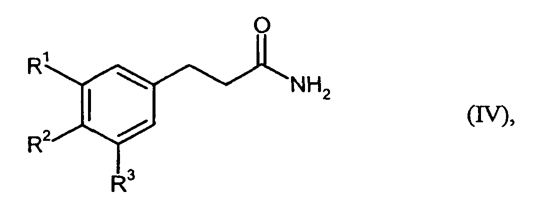 in which
one of the radicals R¹ and R² represents fluorine, trifluoromethyl, trifluoromethoxy, tetrafluoroethoxy or pentafluoroethoxy and the other represents hydrogen or
R¹ and R² together represent -O-CF₂-O-, -O-CF₂-CF₂- or -O-CF₂-CF₂-O- and
R³ represents hydrogen or chlorine,
with the exception of the compounds 3-(3-fluorophenyl)propionamide, 3-(4-fluorophenyl)propionamide, 3-[3-(trifluoromethyl)phenyl]propionamide and 3-[4-(trifluoromethyl)-phenyl]propionamide.

## Revendications

1. Procédé de préparation de phènéthylamines contenant du fluor, de formule I : 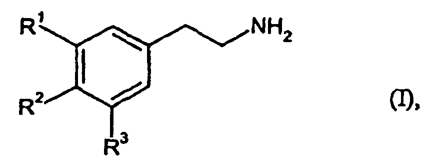 où
parmi les résidus R¹ et R², l'un représente le fluor, le trifluorométhyle, le trifluorométhoxy, le tétrafluoroéthoxy ou le pentafluoroéthoxy et l'autre l'hydrogène, un alkyle en C₁-C₆, un alcoxy en C₁-C₆, le fluor, le trifluorométhyle, le trifluorométhoxy, le tétrafluoroéthoxy ou le pentafluoroéthoxy ou
R¹ et R² ensemble représentent -O-CF₂-O-, -O-CF₂-CF₂- ou -O-CF₂-CF₂-O et
R³ représente l'hydrogène, le chlore, un alkyle en C₁-C₆, ou un alcoxy en C₁-C₆, qui est **caractérisé en ce que** l'on fait réagir, dans une première étape, un bromobenzène substitué de formule 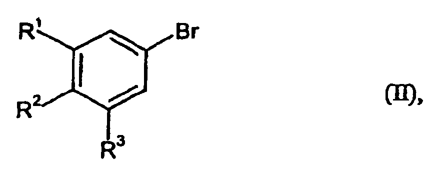 où
R¹, R² et R³ ont la signification indiquée pour la formule (I),
avec de l'acrylamide en présence d'un catalyseur de palladium,
**en ce que** l'on effectue, dans une deuxième étape, une hydrogénation catalytique de l'arylacrylamide obtenu de formule 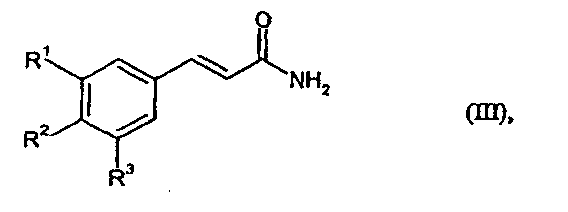 où
les résidus R¹, R² et R³ ont la signification indiquée pour la formule (I),
et
**en ce que** l'on transforme, dans une troisième étape, l'arylamide obtenu dans la deuxième étape, de formule : 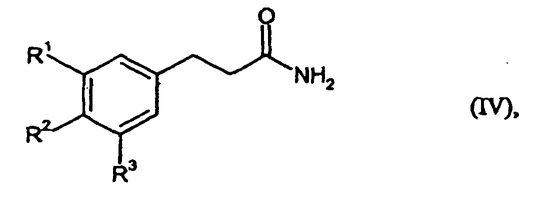 où
R¹, R² et R³ ont la signification indiquée pour la formule (I).

2. Procédé selon la revendication 1, **caractérisé en ce que**, dans les formules (I), (II), (III) et (IV), un des résidus R¹ et R² représente un résidu de la série fluor, trifluorométhyle, trifluorométhoxy, tétrafluoroéthoxy ou pentafluoroéthoxy et l'autre résidu l'hydrogène, ou R¹ et R² ensemble représentent -O-CF₂-O-, -O-CF₂-CF₂- ou -O-CF₂-CF₂-O et R³ représente l'hydrogène ou le chlore.

3. Procédé selon les revendications 1 et 2, **caractérisé en ce que** l'on utilise, à titre de catalyseur de palladium pour la première étape de réaction, des complexes de palladium avec des aryl- ou alkylphosphines comme ligands.

4. Procédé selon l'une des revendications 1 à 3, **caractérisé en ce que** l'on met en oeuvre, dans la première étape de réaction, 0,5 à 2 moles du bromobenzène concerné pour 1 mole d'acrylamide et 0,005 à 20 mmol de catalyseur de palladium par rapport au bromobenzène concerné et que l'on réalise cette étape de procédé à une température de 50 à 180°C.

5. Procédé selon les revendications 1 à 4, **caractérisé en ce que** l'hydrogénation catalytique des arylacrylamides de formule (III) se fait avec de l'hydrogène gazeux et avec des métaux précieux sur matériaux supports à titre de catalyseurs.

6. Procédé selon les revendications 1 à 5, **caractérisé en ce que** l'on réalise la deuxième étape de réaction en présence d'un agent diluant.

7. Procédé selon les revendications 1 à 6, **caractérisé en ce que** l'on utilise dans la deuxième étape de réaction, par mole d'arylacrylamide de formule (III) 0,001 à 0,5 mole de catalyseur (calculé sous forme de métal) et que l'on réalise cette étape de réaction à une température de 0°C à 120°C.

8. Procédé selon les revendications 1 à 7, **caractérisé en ce que** l'on réalise la transformation des arylamides de formule (IV) en phènéthylamines de formule (I) avec une base aqueuse en présence de brome ou de chlore.

9. Arylacrylamides de formule 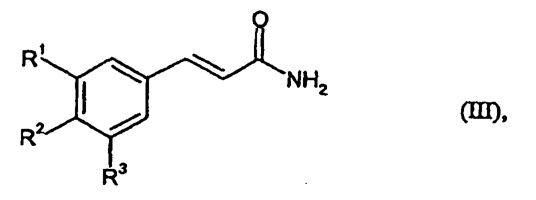 où
parmi les résidus R¹ et R², l'un représente le fluor, le trifluorométhyle, le trifluorométhoxy, le tétrafluoroéthoxy ou le pentafluoroéthoxy et l'autre l'hydrogène, ou
R¹ et R² ensemble représentent -O-CF₂-O-, -O-CF₂-CF₂- ou -O-CF₂-CF₂-O et
R³ représente l'hydrogène ou le chlore,
à l'exception des composés 3-(3-fluorophényl)-acrylamide, 3-[3-(trifluorométhyl)-phényl]-acrylamide et 3-[4-(trifluoro-méthyl)-phényl]-acrylamide.

10. Arylamides de formule 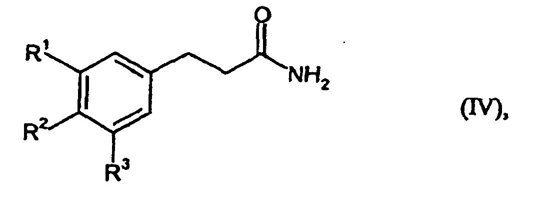 où
parmi les résidus R¹ et R², l'un représente le fluor, le trifluorométhyle, le trifluorométhoxy, le tétrafluoroéthoxy ou le pentafluoroéthoxy et l'autre l'hydrogène, ou
R¹ et R² ensemble représentent -O-CF₂-O-, -O-CF₂-CF₂- ou -O-CF₂-CF₂-O et
R³ représente l'hydrogène ou le chlore,
à l'exception des composés 3-(3-fluorophényl)-propionyl-amide, 3-(4-fluorophényl)-propionylamide, 3-[3-(trifluoro-méthyl)-phényl]-propionylamide et 3-[4-trifluorophényl]-propionylamide.
